# EUROPEAN PATENT SPECIFICATION

(11) **EP 0 565 123 B1**
(45) Date of publication and mention of the grant of the patent: **03.07.1996**
(21) Application number: 93105884.6
(22) Date of filing: 08.04.1993
(51) Int. Cl.: G01J 5/04, A61B 5/00

(54) **Radiation clinical thermometer**
Klinisches Strahlungsthermometer
Thermomètre médical à radiations

(30) Priority: 08.04.1992 JP 86738/92
(43) Date of publication of application: 13.10.1993
(73) Proprietor: OMRON CORPORATION, Kyoto 616 (JP)
(72) Inventor: Makita, Shigeru, c/o Omron Corp., Int. Prop. Cen., Nagaokakyo-City, Kyoto 617 (JP); Sano, Yoshihiko, c/o Omron Corp. Int. Prop. Cen., Nagaokakyo-City, Kyoto 617 (JP); Nakamura, Yasushi, c/o Omron Corp. Int. Prop. Cen., Nagaokakyo-City, Kyoto 617 (JP); Ota, Hiroyuki, c/o Omron Corp. Int. Prop. Cen., Nagaokakyo-City, Kyoto 617 (JP)
(74) Representative: WILHELMS, KILIAN & PARTNER Patentanwälte

(56) References cited:
- EP-A- 0 445 784
- WO-A-92/11800
- US-A- 4 993 424
- US-A- 5 046 482

## Description

### BACKGROUND OF THE INVENTION

### 1. Field of the Invention

This invention relates to a radiation clinical thermometer, and more particularly to an improved radiation clinical thermometer for measuring human body temperature by sensing infrared radiation.

### 2. Discussion of the Related Art

A conventional radiation clinical thermometer includes a body housing enclosing a circuitry and power source, a probe externally projecting from the housing and internally enclosing an infrared radiation sensor (temperature sensor), and a probe switch so as to collect infrared radiation through the probe. An example of such a conventional radiation clinical thermometer is disclosed in U.S. Pat. No. 4,895,164. For the measurement of a body temperature, the conventional radiation clinical thermometer is used by inserting its probe into an ear opening. When the thermometer is not in use, a cap is mounted on the probe. For stains prevention and sanitation, the probe is mounted by a probe cover for use on measuring. WO-A-92/11800 (= EP-A-0 567 646), published 23.07.92, describes such a radiation clinical thermometer which furthermore comprises a slider for removing the probe cover from the tip of the probe.

The conventional clinical thermometer has the disadvantage that unnecessary measurement is initiated upon the depression of a start switch even if any probe cover is not mounted. If a probe cover is mounted on a probe in power-on state of the radiation clinical thermometer designed by these inventors designed to actuate the start switch in response to the movement of probe, the probe moves back as the probe is pushed to the ear opening and start switch is turned to ON for immediate measurement of body temperature. If the probe is unintentionally pushed when cover is not mounted or is going to be mounted in power-on state, however, measurement is initiated to execute unnecessary measurement of body temperature in spite of unmount state of cover. Such initiation of measurement by depressing probe is prevented when the cover is carefully and precisely mounted, but often happens when mounting is roughly executed. If measurement is executed without cover, circuitry is designed to be adjusted in view of attenuation of infrared radiation by cover, thereby displaying the measurement result of a temperature higher than actual temperature (body temperature measured in uncovered state) without providing any precise body temperature measurement. EP-A-0 445 784 describes a radiation clinical thermometer of the type mentioned above which ovecomes this disadvantage by providing a probe cover mount detection switch. However, it does not have a probe cover removing means.

### SUMMARY OF THE INVENTION

It is, therefore, a primary object of this invention to provide an improved radiation clinical thermometer inhibiting measurement when probe cover is not mounted to avoid unnecessary measurement.

According to this invention, there is provided a radiation clinical thermometer, in which a probe cover removable means for removing a probe cover mounted on a probe therefrom is engaged with the probe for a forward-and-backward movement against probe, and a probe cover mount detection switch is disposed to be actuated in response to the movement of the probe cover removable means, whereby any measurement is not initiated even if a body temperature measurement start signal is produced by a start switch when a signal of unmount of probe cover is generated by the probe cover mount detection switch.

According to this radiation clinical thermometer, when probe cover is not mounted on probe, the probe cover mount detection switch (called "cover switch", hereinafter) generates a cover unmount signal in accordance with a position of probe cover removable means so that any body temperature measurement is not initiated in the presence of the cover unmount signal even if start switch is depressed. If cover is mounted, the cover switch generates a cover mount signal according to the position of the probe cover removable means to be ready for measurement so that upon turning on the start switch, body temperature measurement is initiated based on infrared radiation collected from ear opening through probe.

In a radiation clinical thermometer further including a counting means for delaying the initiation of measurement by a predetermined time after release of inhibition of measurement by unmount signal of probe cover, i.e., after a mount signal of probe cover is generated, measurement possible state does not come without elapsing a predetermined time after mounting probe cover, thereby preventing unnecessary measurement by chattering on mounting probe cover to avoid useless measurement just after cover mounting. Moreover, by further employing a construction such that probe is adapted to be for a forward-and-backward movement and the start switch is designed to actuate in response to the movement of probe, probe moves backward by inserting the probe into ear with proper force to turn on the start switch for body temperature measurement.

### BRIEF DESCRIPTION OF THE DRAWINGS

Other objectives and advantages of this invention will be more readily apparent from the following detailed description provided in conjunction with the following figures, of which:
Fig. 1 is an external front view of a radiation clinical thermometer as a preferred embodiment of this invention,
Fig. 2 is an external back view of the thermometer of Fig. 1,
Fig. 3 is illustration for removing a probe cover from the thermometer of Fig. 1,
Fig. 4 is a partial longitudinal sectional view of the thermometer of Fig. 1,
Fig. 5 is a partial cross sectional view of the thermometer of Fig. 1,
Fig. 6 is a flow chart of a body temperature measurement operation of thermometer of Fig. 1,
Fig. 7 is a flow chart of a body temperature measurement operation of thermometer including a counting means,
Fig. 8 is a detailed flow chart of a step ST3 in the flow chart of Fig. 7, and
Fig. 9 is a detailed flow chart of a step ST4 in the flow chart of Fig. 7.

### DETAILED DESCRIPTION OF THE INVENTION

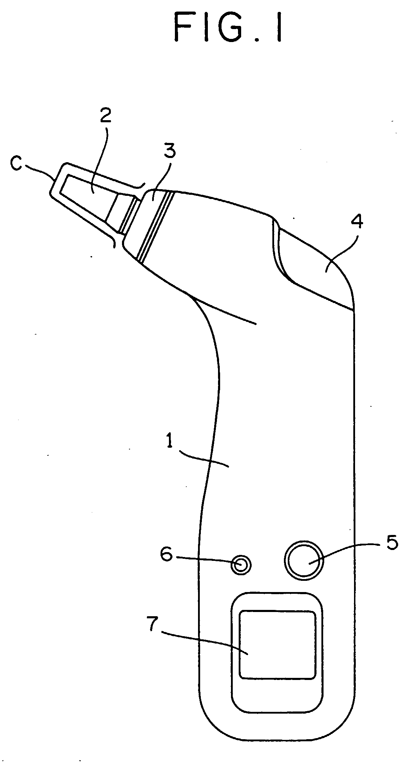

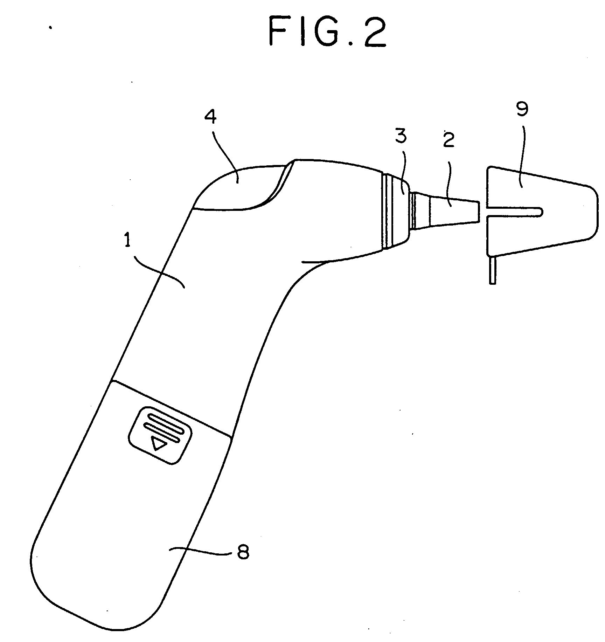

Referring now to Figs. 1 and 2, there are shown front and back external views of a radiation clinical thermometer as a preferred embodiment of this invention. The thermometer includes a body housing 1 enclosing a circuit and power source, a probe 2 enclosing an infrared sensor externally projecting from the housing for a forward-and-backward movement, a release pipe 3 engaged with probe 2 for a forward-and-backward movement, and a release button 4 for pushing out release pipe 3 toward probe, whereby a probe cover removable means is constructed by release pipe 3 and release button 4. On a front wall of the housing 1 of the thermometer there are provided a push button 5 for power source, a push button 6 for illumination, and a LCD display 7. On a rear wall of the housing there is provided a battery cover 8 for replacement of a battery as a power source.

In use of the thermometer to be applied to a human body, probe 2 is so biased externally from housing 1 to slightly move backward toward release pipe 3 by inserting the same to an ear opening with certain force and to move forward to its original position by releasing the certain force. A start switch (probe operation state detection switch, described later) for switching in response to the forward-and-backward movement is so disposed within housing 1 that the start switch is turned to ON when probe 2 moves backward and turned to OFF when a measurement start signal is generated and probe 2 moves forward.

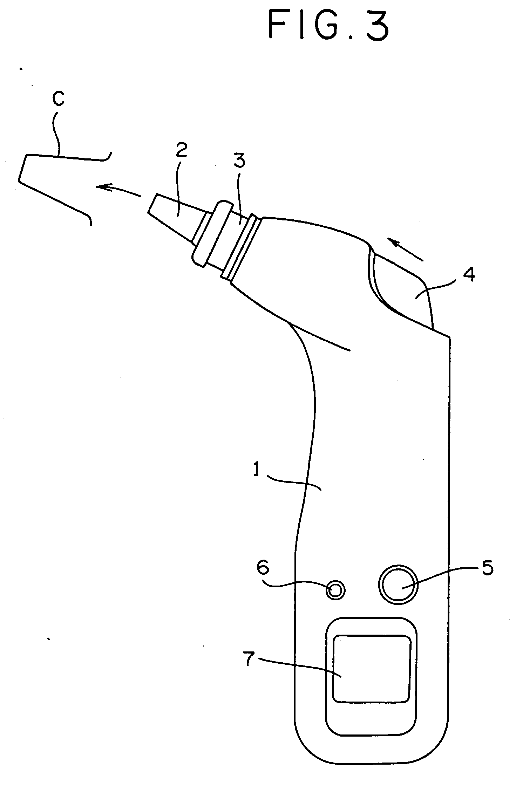

As described above, this thermometer is so designed that in out-of-use mode a cap 9 is mounted on probe 2 and in a measurement mode a probe cover C is mounted on probe 2 to be used for stain prevention and sanitary of probe 2. The probe cover C is adapted to be removed by release pipe 3. By pushing release button 4 in an arrow marked direction shown in Fig. 3, release pipe 3 projects from body housing 1 and cover C mounted on probe 2 is removed from probe 2. Release pipe 3 is locked in a projection state and returned to its original position by releasing the push applied to the release button 4. In order to release the projection of release pipe 3, the pipe 3 has only to be pushed toward body housing 1 for returning to its original position to be locked there. Since pipe 3 in actual use moves backward when the probe cover C is mounted for measurement, intentional push is not required.

Thus, the projection of release pipe 3 shows a state that probe cover C is not mounted on probe 2, and the withdrawal of the pipe shows a state of mounted. Accordingly, a cover switch within housing 1 (probe cover mount detection switch, described later) to be switched in response to the forward-and-backward movement of pipe 3 produces a signal representing whether or not cover C is mounted.

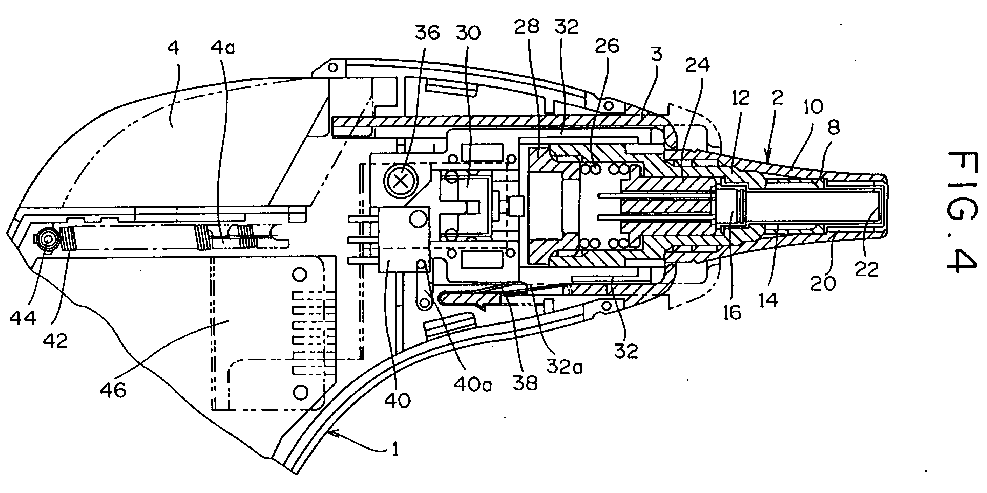

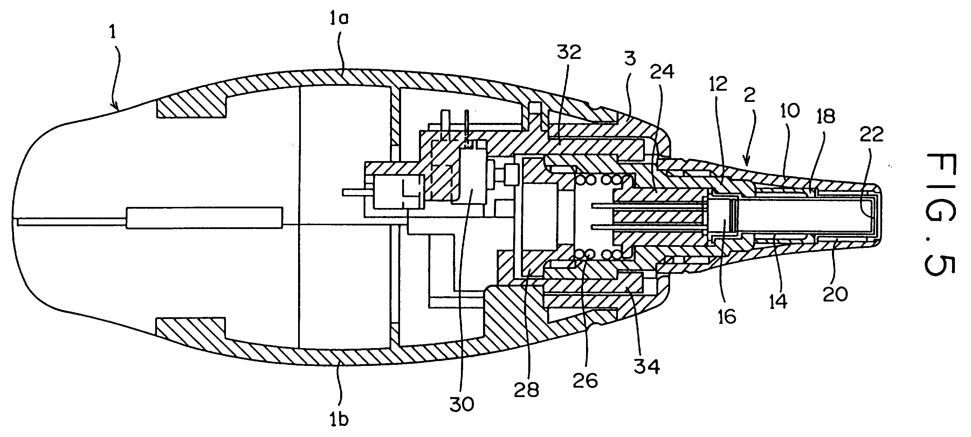

The mechanism of probe 2, release pipe 3, release button 4 and so forth will be described hereinafter in more detail. Returning to Figs. 4 and 5, body housing 1 consists of a front housing la and a ground housing 1b which are separably coupled one after other.

Probe 2 consists of a wrapping member 10 and a probe body 12 removably mounted by wrapping member 10. A wave guide 14 for guiding infrared radiation extends from probe body 12, and an infrared radiation sensor (temperature detection sensor) 16 is disposed at a rear end of guide 14. A wave guide cap 20 is mounted on guide 14 through a ring 18, and at its tip is mounted by a protection filter 22. Infrared radiation passes through protection filter 22 and is guided by wave guide 14 to reach infrared radiation sensor 16.

A lead frame of infrared radiation sensor 16 is pierced thorough sleeve 24 biassed toward wave guide 14 by a spring coil 26 engaged with a rear end of the sleeve, whereby infrared radiation sensor 16 disposed at a front end of sleeve 24 is pushed to a rear end of guide 14 to be held thereby and whole probe 2 is biassed outside housing 1. Spring coil 26 is also engaged with a sensor cap 28 to be supported between sleeve 24 and cap 28. Sensor cap 28 is engaged with a rear end of probe body 12. Probe switch 30 is disposed behind sensor cap 28 to be actuated thereby.

For a closing operation of start switch 30, probe 2 is depressed against spring coil 26 to move backward by a gap of spring coil 26 for moving sensor cap 28 backward. For an opening operation, the force pushing probe 2 is released to return probe 2 to its original position by coil 26, disengaging cap 28 from switch 30.

Start switch 30 is fixed by a tap tight 36 to a probe base 32 mounted on front housing 1a. Probe base 32 is engaged with base cover 34 to produce a chamber inserted by base end of probe 2.

Release pipe 3 engaged with probe 2 (viz. probe base 32 and base cover 34) for a forward-and-backward movement is engaged with a blade spring 38 bending toward probe base 32. Cover switch (probe cover mount detection switch) 40 having a lever 40a moving in response to forward-and-backward movement of release pipe 3 is mounted on probe base 32. In withdrawal state of pipe 3 (probe cover is mounted), blade spring 38 is behind projection 32a formed on base 32 to be locked and lever 40a of cover switch 40 at a rear end of pipe 3 is positioned in a solid line (Fig. 4). In this state (switch 40 is turned to ON), switch 40 generates probe cover mount signal. In a state where pipe 3 projects (cover unmount state), blade spring 38 is in front of projection 32a to lock the state and to release lever 40a to be automatically returned to a position marked by two dotted lines. In this state (switch 40 is OFF), switch 40 generates a cover unmount signal.

Release button 4 for pushing release pipe 3 forward is mounted on body housing 1 for a slidable movement. A pulling spring 42 engaged between an engagement member 4a disposed on button 4 and a pin 44 disposed on front housing 1a biases button 4 backward. As button 4 is pushed against force by spring 42, a tip of button 4 comes into contact with a rear end of release pipe 3 to push the same forward to be locked in a projecting state as described above. If such push is released, button 4 is returned to its original position by spring 42.

A flexible base 46 is wired to electrically connect infrared radiation sensor 16, start switch 30 and cover switch 40 with a circuit board (not shown in drawings), Electronic components are mounted on the circuit board in a predetermined pattern for measurement by thermometer and the board is fixed in front housing 1a of housing 1.

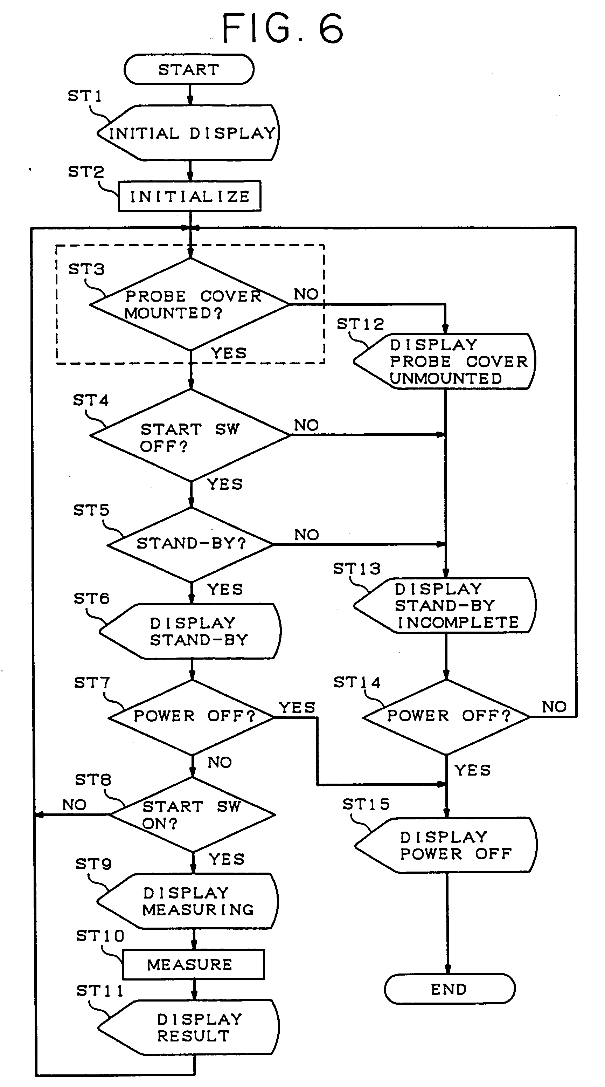

The above-mentioned radiation clinical thermometer is characterized by that any measurement is not initiated even if a body temperature measurement start signal is generated when unmount signal of probe cover is produced by cover switch 40. This control is executed by circuits, and its operation will be explained hereinafter according to a flow chart shown in Fig. 6.

Upon depression of power switch button 5 for power-on, predetermined information is displayed on LCD display 7 of body housing 1 as an initial display in a step 1 (briefly expressed as "ST1" hereinafter), and various values are initialized (ST2). In ST3 it is inquired if probe cover is mounted. As described above this inquiry is performed based on a signal representing whether a cover mount signal is generated when release pipe 3 is withdrawn or a cover unmount signal is generated when it projects.

If the cover unmount signal (NO response) is generated, a state of unmounted probe cover is displayed (ST12) and incompletion of measurement preparation is displayed (ST13). If the power supply is turned to be OFF for stopping measurement (ST14), a state of power-off is displayed (ST15) to finish the measurement. If the power supply is ON in ST14, the sequence returns to ST3.

If the cover mount signal (YES response) is generated in ST3, it is inquired if start switch 30 is OFF (ST4). If it is ON (NO response), measurement preparation is not completed and the sequence moves to ST13. YES response from ST4 is applied to ST5 to inquire if measurement preparation is completed. Unless completed, the sequence moves to ST13. If completed, completion of measurement preparation is displayed (ST6). After the display of completion, it is inquired if power supply is OFF (ST7). If OFF, the sequence moves to ST15. If in power-on state probe 2 moves backward by depression and start switch 30 becomes ON (ST8), body temperature measurement is initiated and a state of measuring is displayed (ST9). Then, for several seconds, measurement is executed (ST10), a measurement result (body temperature value) is displayed upon completion of the measurement (ST11), and the sequence returns to ST3. Even if start switch 30 is OFF in ST8, it returns to ST3.

Thus, any measurement is not initiated even when start switch 30 becomes ON as long as probe cover is not mounted on probe 2, so that measurement in a cover unmount state is prevented.

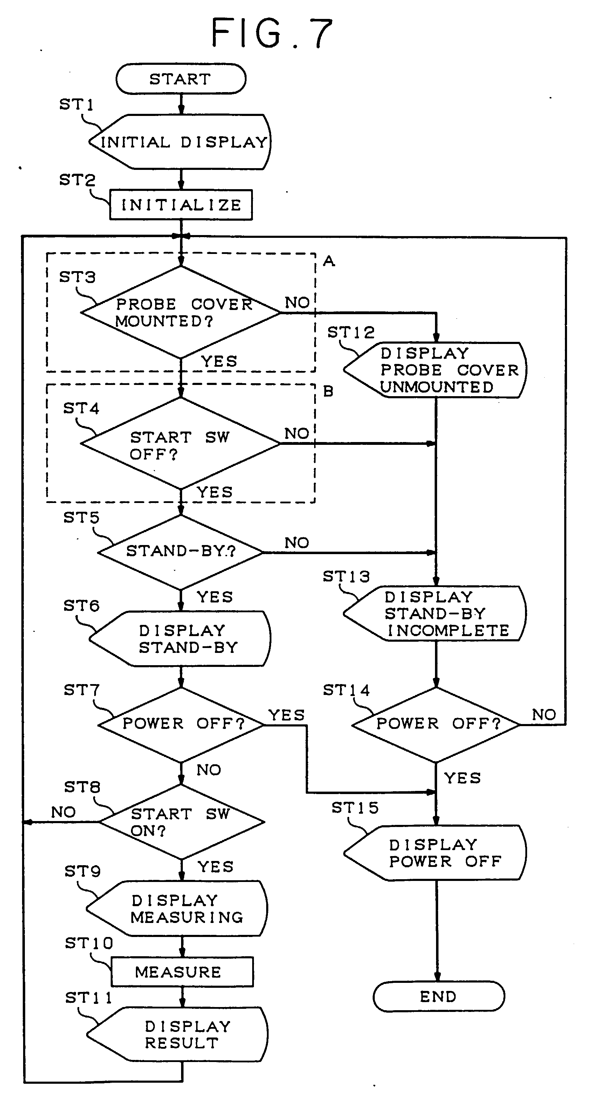

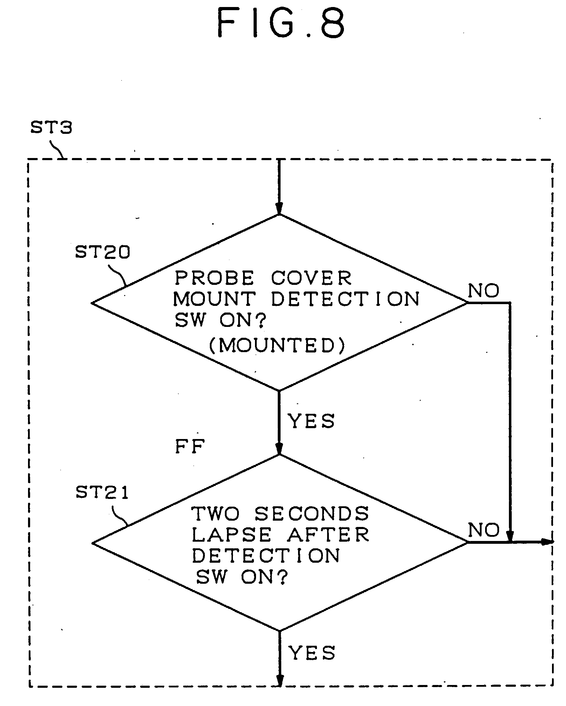

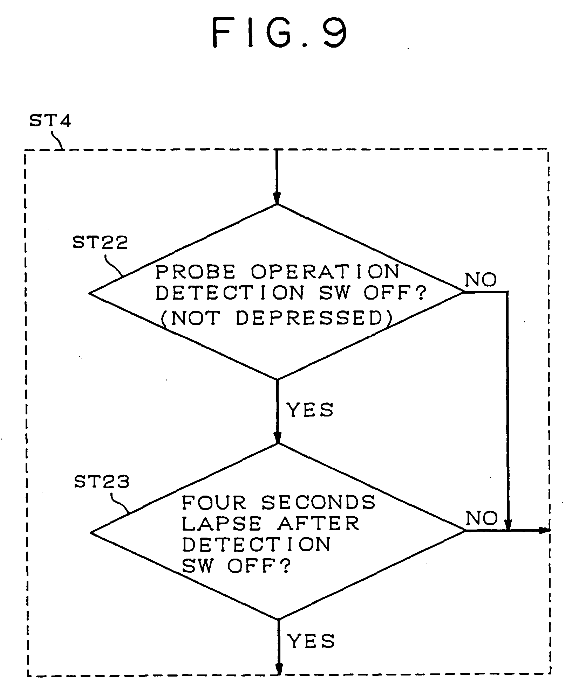

In Fig. 7 there is shown a flow chart when the above mentioned control further includes a counting means for delaying a measurement initiation for a predetermined time is employed after prohibition of measurement initiation by unmount signal of probe cover is released, viz., after the mount signal of probe cover is generated. The flow chart is fundamentally same as that of Fig. 6 except ST3 and ST4 shwon in Figs. 8 and 9.

In ST3 of Fig. 8, when probe cover is mounted in ST20, viz., cover switch (probe cover mount detection switch) 40 is ON, it is inquired if a predetermined time (herein, 2 seconds) has elapsed after switch 40 turns to ON (ST21). If has elapsed, the sequence moves to ST4. If NO response is generated in ST20 and ST21, the sequence moves to ST12 where probe cover unmount is displayed.

In ST4 of Fig. 9, when start switch (probe operation state detection switch) 30 is OFF (probe 2 is not depressed) in ST22, it is inquired if a predetermined time (herein, 4 seconds) has elapsed after start switch 30 turns to OFF (ST22). If YES response is produced, it moves to ST5. If NO response is produced in ST22 and ST23, it moves to ST13 where incompletion of measurement preparation is displayed.

According to flow charts of Figs. 7, 8 and 9, counting is executed for several seconds just after probe cover is mounted or just after probe 2 is once depressed to return to its original state, measurement is prohibited until measurement preparation is completed.

According to the radiation clinical thermometer, unnecessary measurement which is generally apt to happen on mounting probe cover on probe is prevented. Though erroneous measurement is conventionally executed when measurement is executed in unmount state of probe cover, such erroneous measurement can be avoided in this thermometer because measurement is prohibited in unmount state. Moreover, the radiation clinical thermometer having counting means prevents unnecessary measurement by chattering on mounting probe cover, so that rough treatment does not invite unnecessary measurement.

## Claims

1. A radiation clinical thermometer including a body housing (1) enclosing a circuit and a power source, a probe (2) projecting to the external from said body housing (1) enclosing a temperature detection sensor (16) therewithin, and a start switch (30) for generating a temperature measurement start signal, comprising
probe cover removing means (3,4) for removing a probe cover mounted on said probe therefrom, said probe cover removing means being engaged with said probe (2) for a forward-and-backward movement against said probe (2), and
a probe cover mount detection switch (40) actuated in response to the forward-and-backward movement of said probe cover removing means, wherein the circuit is arranged so that any measurement is not initiated even if the temperature measurement start signal is produced by the start switch (30) when a probe cover unmount signal is generated by the probe cover mount detection switch (40).

2. A radiation clinical thermometer according to claim 1 further comprising counting means for delaying a measurement initiation for a predetermined time after releasing inhibition of measurement initiation by said probe cover unmount signal or after a mount signal of probe cover is generated.

3. A radiation clinical thermometer according to claim 1 or claim 2 in which said probe is disposed for a forward-and-backward movement and said start switch is actuated in response to the movement of said probe.

## Patentansprüche

1. Strahlungs-Fieberthermometer mit einem Körpergehäuse (1), welches eine Schaltung und eine Spannungsquelle umschließt, einer aus dem Körpergehäuse (1) nach außen herausragenden Sonde (2), welche einen Temperaturnachweissensor (16) umschließt, und einem Startschalter (30) zur Erzeugung eines Temperaturmessungs-Startsignals, mit
Sondenabdeckungsentfernungsmitteln (3, 4) zum Entfernen einer auf der Sonde angebrachten Sondenabdeckung, wobei die Sondenabdeckungsentferungsmittel mit der Sonde (2) für eine Vorwärts-Rückwärts-Bewegung gegen die Sonde (2) im Eingriff sind, und
einem Sondenabdeckungsanbringungs-Nachweisschalter (40), der durch die Vorwärts-Rückwärts-Bewegung der Sondenabdeckungsentfernungsmittel betätigt wird, wobei die Schaltung so eingerichtet ist, daß, auch wenn das Temperaturmessungs-Startsignal durch den Startschalter (30) erzeugt wird, keine Messung in Gang gesetzt wird, wenn ein Sondenabdekkungsabnahmesignal durch den Sondenabdeckungsanbringungsnachweisschalter (40) erzeugt wird.

2. Strahlungs-Fieberthermometer nach Anspruch 1, welches ferner Zählmittel zum Verzögern einer Messungsingangsetzung um eine bestimmte Zeit nach Lösen der Messungsingangsetzungssperre durch das Sondenabdeckungsabnahmesignal bzw. nach Erzeugung eines Sondenabdeckungsanbringsignals aufweist.

3. Strahlungs-Fieberthermometer nach Anspruch 1 oder 2, bei welchem die Sonde für eine Vorwärts-Rückwärts-Bewegung eingerichtet ist und der Startschalter ansprechend auf die Bewegung der Sonde betätigt wird.

## Revendications

1. Thermomètre médical à radiations comportant un boîtier (1) renfermant un circuit et une source d'énergie, une sonde (2) faisant saillie vers l'extérieur depuis ledit boîtier (1) renfermant à l'intérieur un palpeur de température (16), et un commutateur de signal de départ (30) pour créer un signal de départ de mesure de température, comprenant
des moyens de retrait de couvercle de sonde (3, 4) pour retirer de celle-ci un couvercle de sonde fixé sur ladite sonde, lesdits moyens de retrait de couvercle de sonde étant en prise avec ladite sonde (2) en vue d'un mouvement avant et arrière par rapport à ladite sonde (2) et
un commutateur (40) de détection de couvercle de sonde fixé réagissant en réponse au mouvement avant et arrière desdits moyens de retrait de couvercle de sonde, dans lequel le circuit est agencé de sorte qu'aucune mesure n'est déclenchée, même si le signal de départ de mesure de température est produit par le commutateur de signal de départ (30) quand un signal correspondant à un couvercle de sonde non fixé est créé par le commutateur (40) de détection de couvercle de sonde fixé.

2. Thermomètre médical à radiations selon la revendication 1, comprenant en outre des moyens de comptage pour retarder un déclenchement de mesure pendant un temps prédéterminé après la levée de l'interdiction du déclenchement de mesure par ledit signal correspondant à un couvercle de sonde non fixé ou après la création d'un signal correspondant à un couvercle de sonde fixé.

3. Thermomètre médical à radiations selon la revendication 1, ou la revendication 2, dans lequel ladite sonde est disposée en vue d'un mouvement avant et arrière et dans lequel ledit commutateur de signal de départ réagit en réponse au mouvement de ladite sonde.
